# EUROPEAN PATENT SPECIFICATION

(11) **EP 1 225 845 B1**
(45) Date of publication and mention of the grant of the patent: **31.08.2005**
(21) Application number: 00974024.2
(22) Date of filing: 30.10.2000
(51) Int. Cl.: A61F 2/06

(54) **METHOD OF INSTALLING A STENT IN A SLEEVE**
VERFAHREN ZUM LADEN EINES STENTS IN EINER HÜLSE
PROCEDE SERVANT A PLACER UN EXTENSEUR DANS UNE GAINE DE CONFINEMENT

(30) Priority: 29.10.1999 GB 9925636
(43) Date of publication of application: 31.07.2002
(73) Proprietor: C.R. BARD, INC., New Jersey 07974 (US)
(72) Inventor: VOGEL, Michael, 76149 Karlsruhe (DE); HOFFMANN, Martina, 76297 Stutensee (DE); LOMBARDI, Sylvie, 76227 Karlsruhe (DE); EDELMANN, Andreas, 76135 Karlsruhe (DE); HEINZ, Uwe, 76344 Eggenstein-Leopoldshafen (DE); CUNDY, Sandra, M., Mesa, AZ 85202 (US); DEBB, Debra, A., Mesa, AZ 85202 (US)
(74) Representative: HOFFMANN EITLE
(86) International application number: PCT/US2000/029904
(87) International publication number: WO 2001/035861

(56) References cited:
- WO-A-98/27894
- WO-A-99/62428
- US-A- 5 902 334
- US-A- 6 132 458

## Description

This invention relates to methods for installing a self-expanding stent into a confining sleeve at the distal end of a stent delivery system.

### Background

Among the types of stents that may be placed within a human vessel are those characterized as "self-expanding" stents which are confinable, in a small diameter, in the distal end of a catheter-like delivery device in readiness for advancement to and release within a selected site in the vessel. Such stents may be made of a shaped memory alloy, such as a nickel-titanium alloy. For example, such stents may be made from a cylinder of the alloy with a diameter corresponding to, but usually somewhat less than, the diameter which the stent has in its compressed delivery configuration, that is, when it is confined within a sheath of a stent delivery system, for introduction into the body of the patient, for placement at the desired site within the body. When the stent is placed at the desired location in the vessel, the confining sheath is withdrawn proximally, allowing the compressed stent to expand, from memory, to an expanded configuration in which it supports the wall of the bodily lumen in which it is sited.

In the production process, the starting cylinder may be laser-cut to create a matrix of through openings, and then expanded to expanded diameter. It is then heated to give it a "memory" of this configuration. After that, it is necessary to compress the stent sufficient to introduce it into the sleeve or sheath of the delivery system. The compression and loading of the stent into the sleeve may be performed manually and may involve using a fixture, such as a conical funnel through which the stent is urged to progressively compress it to a smaller diameter in which it can be installed into the sleeve. The stent may be covered by an appropriate graft material, such as expanded polytetrafluoroethylene (ePTFE), defining a stent-graft. Alternately, the graft may be a bare, uncovered stent. Where the compression and loading of the stent into the sleeve involves a number of manual steps, there is significant risk of variation in the manner in which the stent engages and is seated within the sleeve.

One object of the present invention is to provide a stent compression process which will allow the step of installing the stent in its delivery sheath or sleeve to be conducted in a more automated way, which should facilitate the objectives of strict quality control, predictability, and further process improvement. Also among the objects of the invention are to provide a process for controlling the degree of longitudinal force applied to the stent; to accurately and repeatedly position the stent at a predetermined distance from the distal end of the delivery sheath; to support the stent from within during its compression.

In EP-A2-0 657 147, C. R. Bard, Inc. proposes to install a stent graft in a sleeve by a process which involves pushing a leading end of the stent graft into a conical confining funnel, until the leading end of the stent, at the tip of the funnel, at its compressed configuration, engages with radially extending spokes of an implant retention device within the sleeve of the delivery system. The spokes act within the sleeve as an anchor, to prevent undesirable axial movements of the stent graft within the sheath/sleeve, or in deployment of the stent graft from the sheath. As the implant expands on deployment, it moves radially outwardly away from the spokes of the anchor device, thereby automatically releasing the anchor device for withdrawal from the blood vessel.

US-A-5,591,222, Susawa et al includes a disclosure of the use of a wire to draw a polymeric stent into a confining tube, as part of the process of placing the stent over an angioplasty balloon.

US-A-5,649,950 Bourne et al discloses use of conical-shaped lumen for collapsing a prosthetic occluder into a narrow or slender configuration advanceable through the lumen of an introducer sheath. The system is described as a front-end loader and contrasted with the Rashkind prior art rear-end loading delivery system. The prosthesis is held on a locking wire, but is drawn by a plurality of suture lines down the conical confinement surface, in a direction opposite to that in which the wire extends away from the prosthesis.

US-A-5749921, Lenker et al discloses loading a Nitinol® stent into a tube by pulling it down a funnel using a plurality of pulling filaments, each looped around a different one of the apices of the stent mesh at its leading end. During deployment of the stent at a site of surgery, the threads can be used once again to pull the stent back into the tube, should this be required. To release the stent, one end of each loop is cut and the other end is used to pull the thread through the stent apex until the cut end passes through the apex

WO 97/17021 Dereume discloses a device to recover into a lumen a stent previously placed in a bodily lumen. The device deploys a ring of hooks to engage one end of the stent and pull it down a flared end of a lumen of the elongate recovery device. See also WO 2000/02615 and CA 2213291 (Gianotti).

Other methods for installing self-expanding stents into confining sleeves are described in WO 9 827 894 A and US-A-5 902 334, which define the preamble of claim 1.

The manual stent installation process for a nickel-titanium stent also may involve cooling the stent to below room temperature, in a water bath. While this is acceptable for an uncoated metal stent, which can be subsequently sterilized totally, it is less attractive for a coated stent or a stent graft, because of the potential for the prosthesis to carry bacteria and pyrogens, originating in the water bath. Thus, it is another object of the present invention to find a method to install a self-expanding metal stent within the sleeve of a delivery system, in a gaseous rather than liquid environment, but which also offers the potential to control the temperature of the stent being installed, especially to bring it to temperatures below room temperature.

### Summary of the Invention

According to the present invention there is provided a method of installing a stent in a sleeve, as defined in claim 1.

Of note is the concept of gripping or clamping the stent receiving end of the sleeve so that, as the stent is pulled down the lumen of the sleeve, the resulting frictional resistance imposes on the sleeve a tensile axial stress rather than a compressive stress and behind the stent rather than in front of the advance of the stent. This facilitates advance of the stent along the sleeve lumen.

In one useful arrangement, the elongate puller is provided as a wire, and that wire is mounted to a post which serves as the puller holder. The post is fixedly mounted to the base, which extends under the device and provides a substrate for a carriage, which carries the gripper, gripper holder and constrictor. A servo-motor in the carriage serves as the translator, to move the carriage along the base away from the post, thereby to translate the sleeve past the stent, as the stent is held on the wire. A force sensor is interposed between the wire and the post, in order to monitor the tensile force in the wire. This force sensor inputs a data processor which is itself controlling the servo-motor, so that translation can be stopped if the tensile force in the wire rises too high. Conveniently, one or more sensors can be located adjacent the sleeve, at the far end, to monitor the arrival of the stent at the far end, and ensure that the stent is brought to the desired end there.

Characteristic of the invention is the adaptor which is able to engage an end of the stent when the stent is expanded, and also adapted to be released from the stent, when the stent is compressed.

In a preferred arrangement of the adaptor, a circle of hooks is provided, each of these hooks being on a long stem running hack to the elongate puller. Each of the hooks can be provided with a bight which has a radially outward-facing opening, so that the circle of hooks can be introduced inside the rim of the leading end of the stent, following which, the outward opening bight of each hook is passed through respective apertures in the cylindrical envelope of the stent, so that the point of each hook lies outside the cylindrical envelope of the stent. The circle of hooks is arranged at evenly-spaced intervals around the circumference of the leading end of the stent. In a typical laser cut cylindrical shape memory alloy stent, in its expanded configuration, there are rhombic apertures in the cylindrical envelope of the stent, and thus a circle of rhombi at the leading end of the stent. Conveniently, there is a hook corresponding to each of the rhombi in the circle of the leading end of the stent. However, one might also provide half as many hooks as there are rhombi, or some other arrangement, so long as the circle of hooks is sufficiently numerous to impose an even pull around the circumference of the leading end of the stent.

In another arrangement, the hooks can be provided as a ball on the end of each stem. With the stem in tension, and the ball radially outside the cylindrical stent envelope, the rhombus vertex catches the ball and prevents the ball passing radially inwardly through the stent envelope, until the tension on the wire is released.

Once the stent has been pulled into the sleeve, the adaptor has to be detached from the leading end of the stent. Conveniently, the structure of the adaptor has enough column strength to allow it to be pushed towards the stent, so that the circle of hooks is itself urged towards the trailing end of the stent, thereby freeing the bight of each hook from the respective vertex of the leading end rhombus with which it is engaged. With the bight of the hook now spaced away from the rhombus vertex at the leading end of the stent, and somewhere in the middle of the rhombic space through which it passes through the cylindrical envelope of the stent, a collar surrounding the circle of stems of the hooks can be urged towards the trailing end of the stent. With the diameter of the collar sufficiently small, the stems are urged radially inwardly, themselves bringing the circle of hooks to a circular envelope with a diameter smaller than the inside diameter of the compressed stent wall. In this configuration, with the hooks no longer penetrating into the cylindrical envelope of the stent, but instead lying entirely radially within it, the way is clear for the circle of hooks to be withdrawn, axially away from the trailing end of the stent, past the leading end of the stent, and out of the sleeve. Preferably, the collar has a leading end rim which passes into the open bight of each of the hooks, and fills the bight, thereby denying any other structure the possibility of falling into the bight of each hook, as the circle of hooks is withdrawn past the leading end of the stent.

For example, each stem could end in a loop instead of a hook, and a purse string could be threaded through apertures of the stent as well as each of said loops. Release of such an adaptor is effected by pulling out the purse string. In such an embodiment, the stems need not be resilient and no surrounding collar is required.

In another arrangement, a plurality of pull strings or wires are used each being threaded through two apices of a stent matrix at the leading end of the stent, these two apices being at opposite ends of a diameter across the stent lumen. Two, three, four or more of such strings can be arranged across different diameters. On the axis, where all strings cross, a single retraction string can be looped around all the pull strings, and can lead out through the stent past the opposite, trailing end of the stent, to pull out the pull strings when their work is done.

Alternatively, each pull string could have its own release string. Each pull string could pass through only one stent apex, or more than two apices.

An adaptor of another form entirely could be used. For example, one resembling a bottle brush might be feasible. In such an adaptor a structure along the axis of the device has a plurality of push strands which are arranged around the circumference of the axis and are resistant to longitudinal compressive stress. One end of each strand is fixed to the axial structure and the other is cantilevered radially outside it. With the fixed ends closer to the trailing end of the stent, pulling on the axial structure from in front of the leading end of the stent brings the cantilevered ends of the strands into contact with push surfaces of the stent, such as apices of its mesh. Continued pulling advances the stent by virtue of the push given to its push surfaces by the strands, now in axial compression. When it is time to release the adaptor from the stent, it can simply be urged rearwardly, past the trailing end of the stent, and the strands slip past the luminal-facing surfaces of the stent.

In a variant, the push strands could be arranged as the ribs of an umbrella with its apex remote from the leading end of the stent and its ribs engaging the stent near its leading end.

For release of such a device, one could either pull on a release string from behind the trailing end of the stent, or push on a push shaft from in front of the leading end of the stent. Another possibility would be to use a ring which embraces the push strands, sliding the ring towards the cantilevered ends to draw them radially inwardly out of engagement with the stent.

An adapter alternately may have the general form of a chuck, which grips the leading end of the stent between two co-axial annular members, which move axially towards each other to grip the stent and away from each other to release the stent.

It will be appreciated that in the presently preferred embodiment of loading device the location of the stent relative to the base remains constant, and the translator moves the sleeve relative to the base, until the stent is in the desired location within the sleeve. However, in alternative embodiments, it could be arranged that the sleeve does not move relative to the base, but the stent is translated, relative to the base as well as the sleeve.

The invention is particularly well suited to the installation of stents which are otherwise not well adapted to installation under water in a sleeve. Thus, it is well adapted to installing encapsulated or covered stents, but can also be used to install bare stents.

The invention is particularly attractive in respect of stents which are themselves sleeved or encapsulated. With the stent being pulled from its leading end, there is friction acting between the stent and the surfaces which are compressing it, leading to a tensile stress within the length of the stent. Some stents are not well adapted to withstand such longitudinal tensile stresses, but sleeved or encapsulated stents are often better able to resist such tensile stresses, because the encapsulation or sleeve can share the longitudinal tensile stresses imposed on the stent.

In one combination, the stent is one which carries a sleeve of expanded PTFE (ePTFE), which is fixed to the metallic cylinder of the stent at zones corresponding to the leading and trailing ends of the stent, but not over the middle part of the length of the stent. One way of achieving this result is to place bands of ePTFE inside the cylindrical envelope of the stent, one at each end of the stent, on a mandrel which then receives the stent and the abluminal ePTFE sleeve. The assembly is sintered, causing the sleeve and bands to bond together, through the apertures of the stent cylinder, at each end of the stent. The ePTFE sleeve is relatively inextensible, and therefore supports the metal stent cylinder against elastic and plastic deformation which might otherwise occur under the tensile stresses imposed on the stent as it is drawn into the sleeve.

As to the loading of the stent into a sleeve, the sleeve is itself elastically deformable, and, in one loading technique, will be subject to tensile stresses during the stent installation process. These forces will produce tensile strain in the sleeve. Unless allowance is made for this strain in the installation operation, then the actual position of the stent in the sleeve will not correspond to the desired position. To compensate for the effect of strain in the sleeve, the speed of translation is reduced as the stent approaches the desired end point of its travel within the sleeve. A laser beam extending across the path of the sleeve can serve to detect the presence or absence of the sleeve, thereby detecting whether the sleeve is still strained and, in effect, establishing whether the actual position of the far end surface of the sleeve corresponds to the desired location. A magnetic inductance sensor detects the arrival of the stent being translated with the sleeve at the desired location near the distal end of the sleeve. Data from these two sensors, delivered to the data processor, allow the data processor to terminate the pulling process at a point which places the stent at a desired distance from the far end surface of the sleeve.

For a better understanding of the present invention, and to show more clearly how the same may be carried into effect, reference will now be made, by way of example, to the accompanying drawings.

### Brief Description of the Drawings

Figure 1 is a longitudinal diametrical section through the stent, adaptor, gripper and constrictor of the device of the present invention;
Figure 2 is a longitudinal diametrical section through the adaptor of the device, showing its internal structure;
Figure 3 shows a sleeved stent with a leading end engaged by the adaptor;
Figure 4 is a longitudinal section through a first embodiment of the device of the present invention, showing it in an initial configuration; and
Figure 5 is a section corresponding to that of Figure 4, showing the device in a final configuration.
Figure 6 is a longitudinal diametrical section of a second embodiment of stent loading machine engaging a stent which has a plurality of beads arranged around its leading end;
Figure 7 is a section as in Figure 6, showing a later stage in the loading of the stent;
Figure 8 is a diagrammatic illustration of a stent threaded with wires to facilitate drawing of the stent through a constricting device to compress the stent and lead it into an end of the sleeve;
Figure 9 is a highly diagrammatic illustration of the end of the stent as shown in Figure 8, as seen from the right end of Figure 8;
Figure 10 is a diagrammatic illustration of the apparatus for supporting the sleeve as the stent is drawn through the constrictor and into the sleeve; and
Figure 11 is an illustration of the enlarged open end of the constrictor showing a stent partially drawn into the constrictor, the stent having been reinforced with an internal support tube.

### Detailed Description

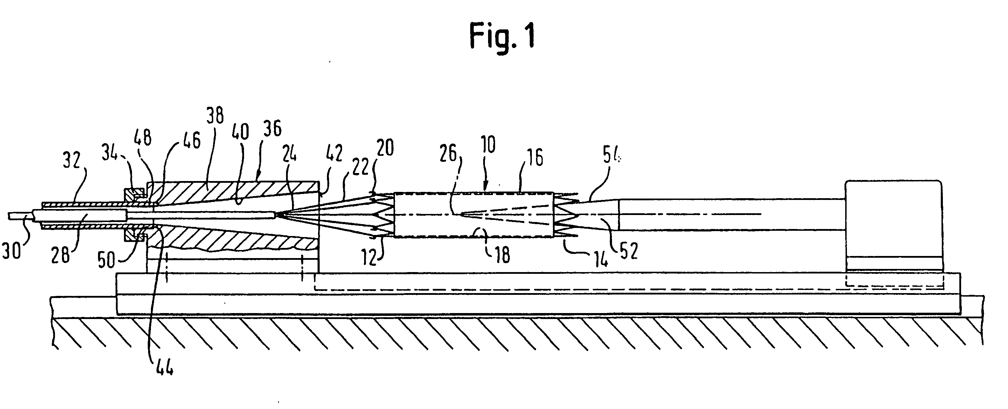

Referring first to Figure 1, there is shown a stent 10 in its expanded configuration, and with a leading end 12 and a trailing end 14, an abluminal surface 16 and a luminal surface 18. A circle of hooks 20 engages the leading end 12 of the stent. Each of the hooks 20 has a long stem 22, and all of these stems extend to a root portion 24 located close to a long axis 26 of the device in general. Over the circle of root portions 24 of the stems 22 is a collar 28 which is slidable over the stems 22, up to the hooks 20.

In other embodiments, detent surfaces other than hooks could be used. For example, a little ball of the end of each stem 22 might be effective, depending on the design of the locations in the stent where the stems are to be coupled.

An elongate puller in the form of a wire 30 is attached to the portions 24 of the stems 22 and extends through and beyond the collar 28. The structure of hooks 20, stems 22, and collar 28 constitute an adaptor 41 which couples the trailing end 43 of the pulling wire 30 to the leading end of the stent 10.

The sleeve 32 into which the stent is to be loaded is supported to receive the pulling wire 30 that extends through the lumen 33 of the sleeve. In the embodiment shown in FIGS. 1-5, a receiving end 50 of the sleeve 32 is gripped in a gripper chuck 34. The chuck 34 is in fixed position or relationship to a constrictor 36 which is in the form of a block 38 of large thermal capacity, which itself defines a tapered, conveniently conical, constricting surface 40 with a wide end 42 and a narrow end 44. At this narrow end 44 is a shoulder surface 46 at the end of a bore surface 48 which snugly receives the receiving end 50 of the sleeve 32. The sleeve has a long axis corresponding to the axis 26 of the device in general. As will be described, the stent will be compressed and drawn into and through the sleeve 32 until the stent is located in a predetermined position at the opposite end of the sleeve. The receiving end 50 of the sleeve thus will serve as the proximal end of the delivery device and the opposite end of the sleeve will serve as the distal end to be inserted into the patient. When the stent is positioned in the sleeve in readiness for deployment in the patient, it will be located near the distal end of the sleeve.

Also to be seen in Figure 1 is a mandrel 52 which has a tapered conical surface 54 which complements the tapered conical surface 40 of the constrictor block 38, so that advancement of the mandrel 52 along the axis 26, into the conical cavity of the constrictor block 38 leads to the definition of a conical annular space through which the hook stems 22 extend.

Conceivably, the constrictor could oscillate axially to assist advance of the stent down to its narrow end. If so, it may be helpful to provide the taper surfaces with a "one-way" structure of steps and terraces, like the underside of a cross-country ski, so that the constrictor can slip backwards relatively easily but on its forward stroke towards the leading end of the stent, tends to carry the stent with it. Alternatively or additionally the cone could be coated with a slippery material to assist stent advance.

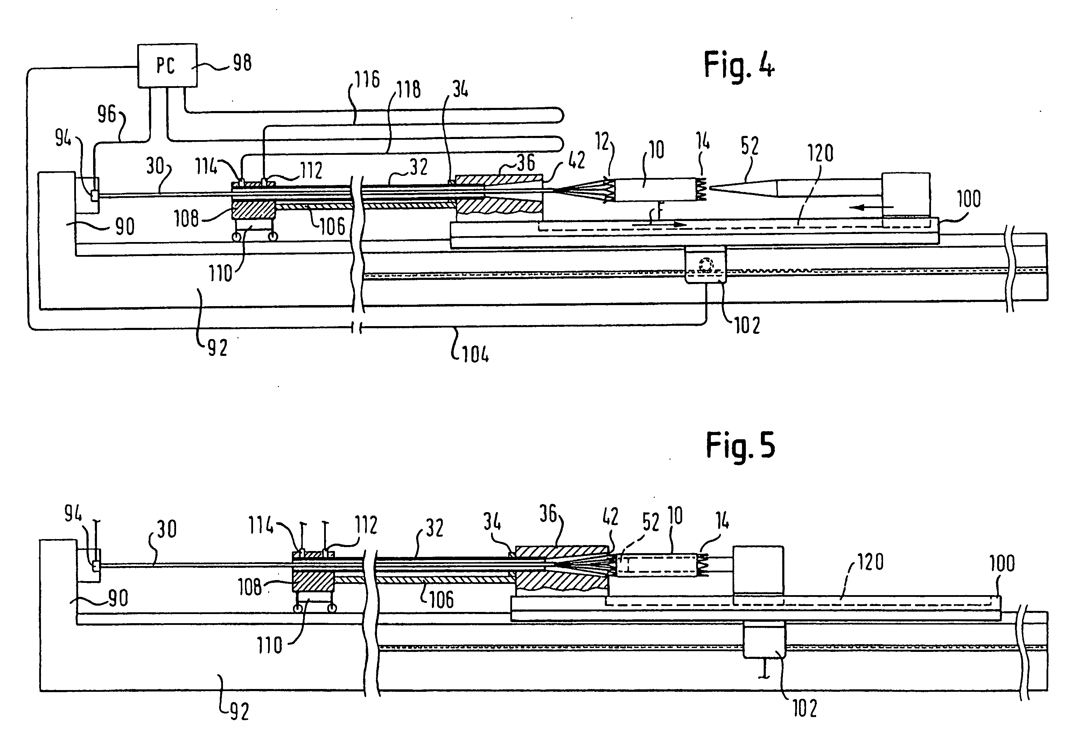

Not shown in Figure 1, for reasons of clarity, are the details of the surrounding structure of the device. (For this, see Figures 4 and 5 and the description below, in relation to those figures).

In operation, a carriage which carries the gripper chuck 34 and constrictor 36 and mandrel 52 is caused to move to the right as shown in Figure 1, while the pulling wire 30 is held fast. As will be appreciated, this has the effect of drawing the hooks 20 down the annular space adjacent the tapered conical surface 40 of the block 38, and the hooks carry with them the leading end 12 of the stent 10. Thus, progressively, the full length of the stent 10 is drawn down the cone and into the receiving end 50 of the sleeve 32. Further progress of the carriage to the right in Figure 1 pulls the stent 10 along the full length of the sleeve 32.

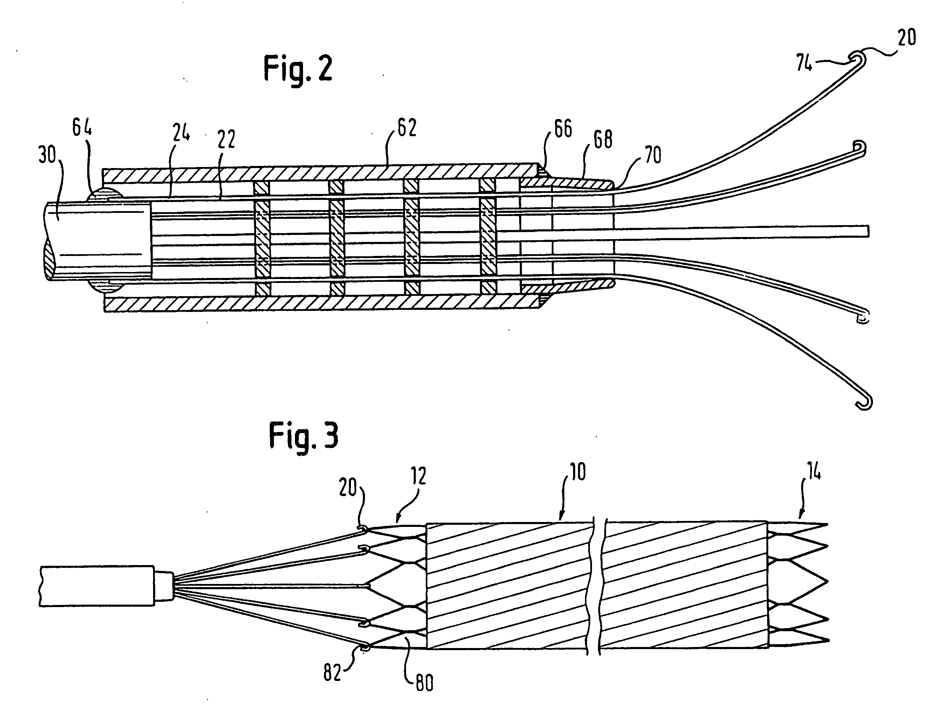

Turning now to Figure 2, the internal construction of the adaptor is visible. A welding bead of fillet 64 fixes the root zones 24 of the stems 22 to the pulling wire 30. The stems 22 are surrounded by a moving collar 62. The fixed sleeve 60 is attached by a welding bead 64 to the pulling wire 30. Attached to the moving collar 62, by a further welding bead 66, is a collar ring 68 having a leading end rim '70. The outside diameter of the collar ring 68 is somewhat smaller than the outside diameter of the moving collar 62. Inside the moving collar 62 is a plurality of apertured rings 72. In the present embodiment, there are twelve hooks 20 and each of the rings 72 has a circle of 12 apertures, each to receive a different one of the stems 22. These apertured rings '72 serve to maintain the spaced even circular arrangement of the long stems 22 and help the stems resist buckling in response to a compressive load.

In use, the moving collar 62 is brought to a proximal location as shown in Figure 2. The stems 22 are provided with a relaxed disposition in which they curve radially outwardly, so that the hooks 20 are on a circular envelope of relatively large diameter. Sliding the collar 62 to the right hand side in Figure 2 brings the rim 70 of the collar ring 68 into the bight 74 of each of the hooks 20. The stems 72 deform elastically, and the circle on which the hooks 20 is located is now a circle of radius corresponding to the radius of the collar ring 68. This is small enough, relative to the inside diameter of the stent cylinder, to allow the hooks 20 to pass along the cylindrical cavity within the stent, without engaging on any part of the apertured wall of the stent, and the presence of the collar ring 68 inside the bight 74 of each hook itself prevents any other structure from passing into the bight of the hook.

Figure 3 shows the circle of hooks 20 each engaged with a different rhombic aperture 80 at the leading end of the stent 10. In fact, each rhombic aperture has a vertex 82 closest to, the leading end 12 of the stent 10 and it is with this vertex 82 that each hook 20 is engaged, for pulling the stent 10 into the sleeve 32.

For coupling the hooks 20 to the stent 10, the collar 62 is slid tip to the hooks 20 and then the circle of hooks 20 is introduced into the leading end of the stent, to bring each of the hooks 20 into a position more or less central within its corresponding rhombic aperture 80. Then the collar 62 is slid proximally back towards the Figure 2 position, allowing the hooks 20 to expand radially outwardly by resilient elastic deformation of the stems 22, until each hook 20 penetrates through the cylindrical envelope of the stent 10 at one of the rhombic apertures 80, with each of the twelve hooks, 20 extending through a different one of the circle of twelve rhombic apertures 80 at the leading end of the stent 10.

Reverting again to Figure 1, the sleeve 32 destined to receive the stent 10 is gripped in chuck 34 and the wire 30 is, then passed through the constrictor block 38 and sleeve 32, until its leading end 39 can be made fast to a puller holder, ready for pulling the stent 10 into the sleeve 32.

Now looking at Figure 4, one sees the leading end 39 of the pulling wire 30 made fast to a post 90 on a base 92 and with a force sensor 94 in the post 90 and arranged to monitor tensile force in the pulling wire 30. The sensor 94 inputs, along lead 96, a data processor 98.

On the base 92 is a carriage 100 mounted for linear translational movement towards and away from the post 90. Within the carriage 100 is a servo motor 102 controlled, via lead 104, by the data processor 98. The carriage 100 carries the constrictor 36, mandrel 52 and sleeve gripper 34. Further, the sleeve 32 is supported from below on a beam 106 which extends from the carriage 100 the full length of the sleeve 32, up to a peninsular block 108 supported above the base 92 by an undercarriage 110. The peninsular block 108 travels with the carriage 100, when the servo motor 102 is actuated.

Mounted on the peninsular block 108, which supports the far end of the sleeve 32, is a plurality of sensor elements, in particular, an optical sensor 112 and a magnetic induction sensor 114. The inductive sensor 114 detects the arrival, at the distal 37 of the sleeve 32, of the stent 10, and to signal that arrival to the data processor 98, through lead 118. The optical sensor 112 passes a laser beam across the locus of the sleeve 32 to sense the end of the sleeve and inform the data processor through lead 116, so that the stent 10 can be brought to a precisely specified location in the sleeve 32 relative to the sleeve end. The sleeve 32 is fixed against movement relative to the peninsular block 108, but only at its receiving (proximal) end 50, so that tensile stresses in the sleeve 32 can alter the position of its distal 37 relative to the sensor 112. Provision of sensor 112 therefore can inform the data processor when the tensile strain in the sleeve 32 has fallen below a specified low value, corresponding to arrival of the stent at the desired end position, and enabling the pulling process to be terminated to lace the stent at just the correct desired distance from the distal end of the sleeve. Readers will appreciate that elastic deformation of the synthetic polymeric materials of stent sleeves is a time-dependent phenomenon, so time has to be allowed, at the end of the pulling process, for the sleeve to relax into its unstressed length.

Not shown in Figure 4 are the arrangements for temperature control of the block 38 of the constrictor 36. Those skilled in the art will not find it difficult to install arrangements to bring the block 38 to the temperature prescribed for optimum installation of the stent.

Figure 4 shows the device before stent installation begins, and Figure 5 shows the device once stent installation is complete. With reference to Figures 4 and 5, the stent installation process proceeds as follows.

With the pulling wire coupled to the stent, and installed as described above with reference to Figure 1, the carriage 100 is moved to the right in Figure 4, so as to bring the leading end 12 of the stent 10 to the wide end 42 of the conical constricting surface 40. The mandrel 52 is advanced to the left in Figure 4, along a key way 120 until it fits within the block 38 to complement the block conical surface 40. Further translation of the carriage 100, in the direction of arrow F, has the effect of carrying the constrictor block 36 along the length of the stent 10, from the leading end 12 towards the trailing end 14, so that the length of the stent is drawn down the conical surface 40 and into the receiving end 50 of the sleeve 32. Should at any time the tensile stress in the pulling wire 30 exceed a pre-determined limit, as measured by force detector 94, then the data processor 98 arrests movement of the carriage by terminating the drive power to the servo motor 102.

As drawing of the stent 10 along the sleeve 32 proceeds, so the receiving end of the sleeve experiences a tensile stress. Note, however, that the sleeve ahead of the stent is not under any axial stress so that stresses in the sleeve at the leading end of the stent are at a minimum, facilitating progress of the stent along the lumen of the sleeve. Effectively, with the sleeve in tension, one is pulling a sleeve over the stent, as opposed to pushing a sleeve over the stent. Towards the end of the pulling process, as shown in Figure 5, substantially the full length of the sleeve 32 is under tensile stress, leading to an increase in its length. However, as explained above, the information which the data processor receives from sensors 112, 114 allows it to translate the sleeve 32 to a position relative to the stent 10 which sets the stent 10 at exactly the desired distance from the end surface of the sleeve 32. At that point, further translation of the carriage 100 is stopped, and the installation process is complete. At: this point, the device can be switched off, and the sleeve 32 removed from it, with the stent 10 in place, yet still connected by the adaptor hook ring to the pulling wire 30.

Reverting now to what is shown in Figures 2 and 3, it will be appreciated that, to remove the circle of hooks 20 from the installed stent 10, the tension has to be taken off the pulling wire 30, and the hooks 20 allowed to relax into the center of the rhombic apertures of the stent 10 in which they are engaged. Then, a movement of the collar 62, up to the hooks 20, has the effect of bringing the rim 70 of the collar ring 68 into the bight 74 of each of the hooks 20, and bringing the diameter of the circular hooks 20 down to a diameter small enough to allow all of the hooks to be drawn out from the cylindrical cavity of the stent, without fouling the cylindrical envelope of the stent.

At this point, the stent and sleeve stand alone, and can be passed forward for the further steps of the process of constructing the stent delivery system.

Important to note is that the distal end of the sleeve is not engaged mechanically by any component of the loading system. The end can therefore be polished in an earlier process step, and does not need to be cut or polished after loading. This is important, because the steps of cutting and polishing could conceivably give rise to the undesirable presence of loose particles inside the sleeve at its distal end. The other end of the sleeve, which has been held in the gripper chuck 34, is the proximal end of the sleeve in use. This can be cut, if necessary, after the stent has been loaded.

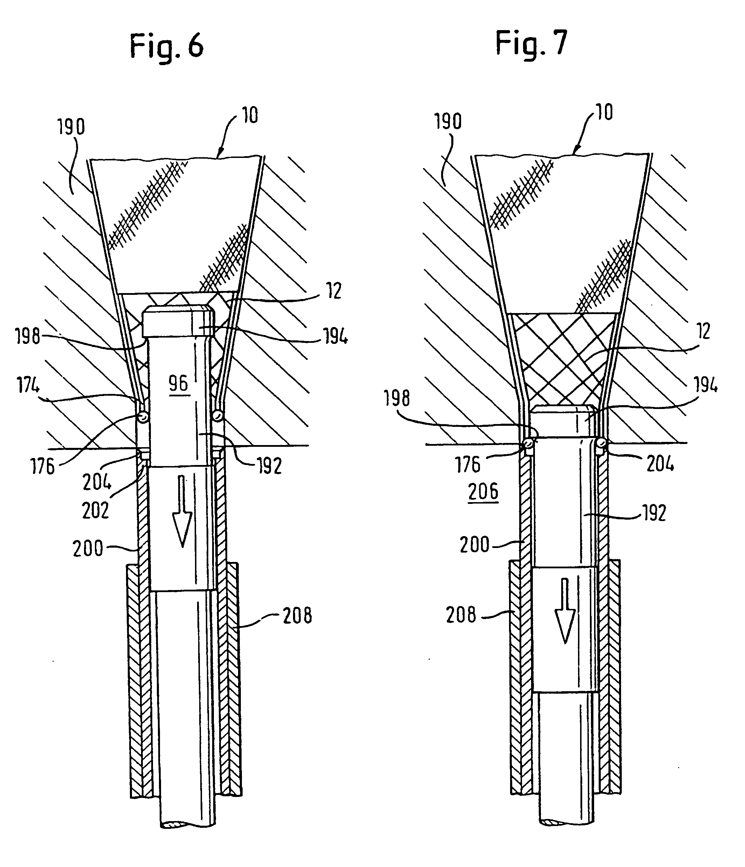

Figure. 6 shows a leading end portion of another embodiment of stent 112. At the end vertex of each cell in the first circumferential ring of cells of the stent, the material of the stent matrix is continued into an extending portion 174 with a width comparable to its thickness dimension so that, in cross-section, it is more or less square. On each such square section spigot 174 is mounted a spherical Nitinol bead 176 which has a through bore on a diameter of the bead, to receive the spigot 174. The Nitinol bead 176 is welded to the spigot 174. It will be appreciated that, by virtue of the rounded surface and greater thickness of the sphere 176 relative to the struts 154, the free vertices defining the end of the stent, and the end of each cell in the end ring of cells of the stent, is less likely to cause trauma in the bodily tissue in which the end vertices is embedded, than if the spheres 176 were absent.

Furthermore, as shown in Fig. 6, the ring of beads 176 brings advantages when it comes to loading the stent onto a delivery system, and keeping control of the stent while the stent is being deployed into the body from the delivery system. Specifically, the ring of relatively thick beads 176 provides a point of purchase for gripping surfaces to impose forces on the stent, while it is being loaded into a delivery system, and while it is being deployed from that delivery system. In one example, the beads 176 could be gripped between circumferential surfaces, one inside the stent annulus and one outside the stent annulus, with a spacing between such co-axial surfaces which is wide enough to receive the thickness of the stent matrix, but does grip the spheres 176 on each side of the thickness of the stent matrix.

Viewing Figures 6 and 7, the stent 112 is shown schematically within the truncated cone of a loading mandrel 190, with its 1eading end at the narrow end of the cone, tipped by the beads 176. Within the leading end of the stent is a loading rod 192 with a somewhat larger diameter head 194, the transition from the head 194 to the cylindrical portion 196 of the rod 192 being accomplished by an arcuate shoulder surface 198. The concave outer surface of the shoulder 198 has a curvature which corresponds to the curvature of the beads 176.

Beyond the narrow end of the truncated cone 190 is a gripping sleeve 200 which has at its gripping end 202 an arcuate gripping shoulder 204, also having a curvature corresponding to that of the spherical surface of the bead 176.

As can be seen from Figure 7, drawing the gripping rod 192 down on to the beads 176 achieves an entrapment of the beads 1'76 in an annulus defined by the gripping shoulders 198 and 204. With the position of the gripping rod 192 maintained close to the gripping end 202 of the gripping sleeve 200, further pulling down of the gripping rod 192, away from the truncated cone 190, permits the advancement of the stent 112 into the cylindrical space shown in Figure 7, within the block 206.

The block 206 receives a sleeve 208 in which the stent 112 is to be housed, in a delivery system for placing the stent 112 at a desired location within the body, for location, a catheter. Continued downward pulling on the gripper rod 192, beyond the position shown in Figure 7, can carry the stent 112 fully inside the sleeve 208 of the catheter delivery system. Once the stent 112 is within the sleeve, the gripping sleeve 200 can be withdrawn forwardly, i.e. downwardly in the Figure 7 view, while the gripper rod 192 can be withdrawn rearwardly from the stent, i.e. upwardly as shown in Figure 7 and back past the trailing end of the stent. Alternatively, once the gripping sleeve 200 is withdrawn, it may be possible to withdraw the gripper rod 192 forwardly, given a degree of resilience in the sleeve 208 to allow the enlarged head 194 to slide past the beads 176 at the leading end of the stent.

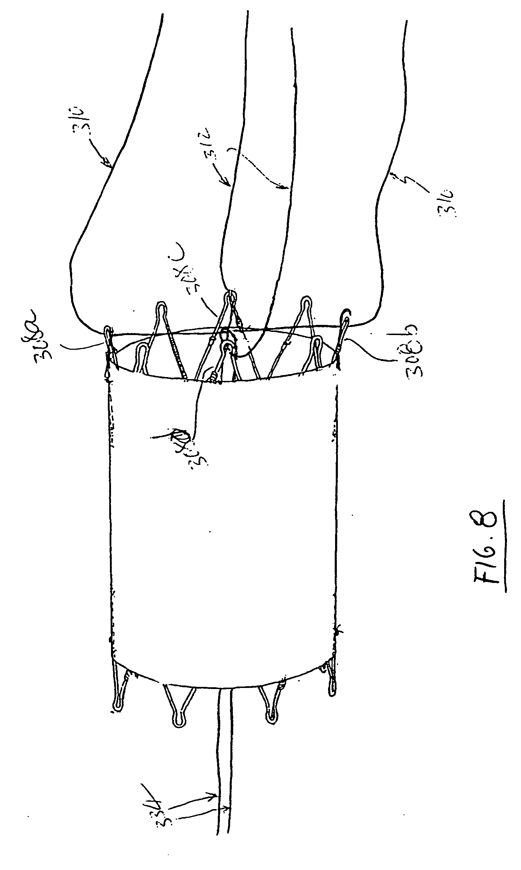

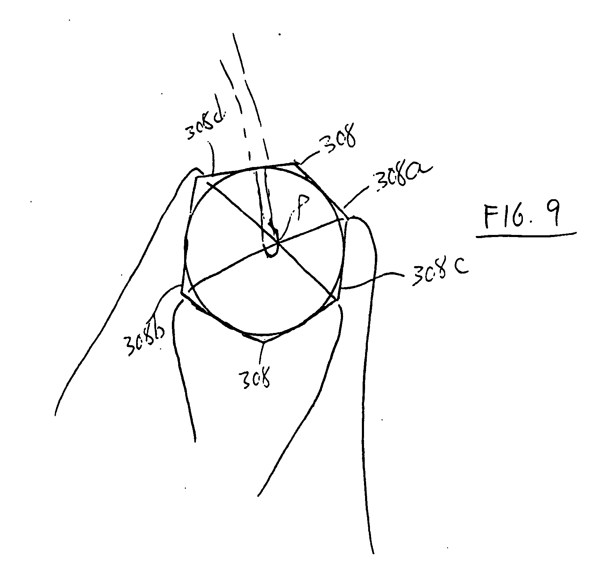

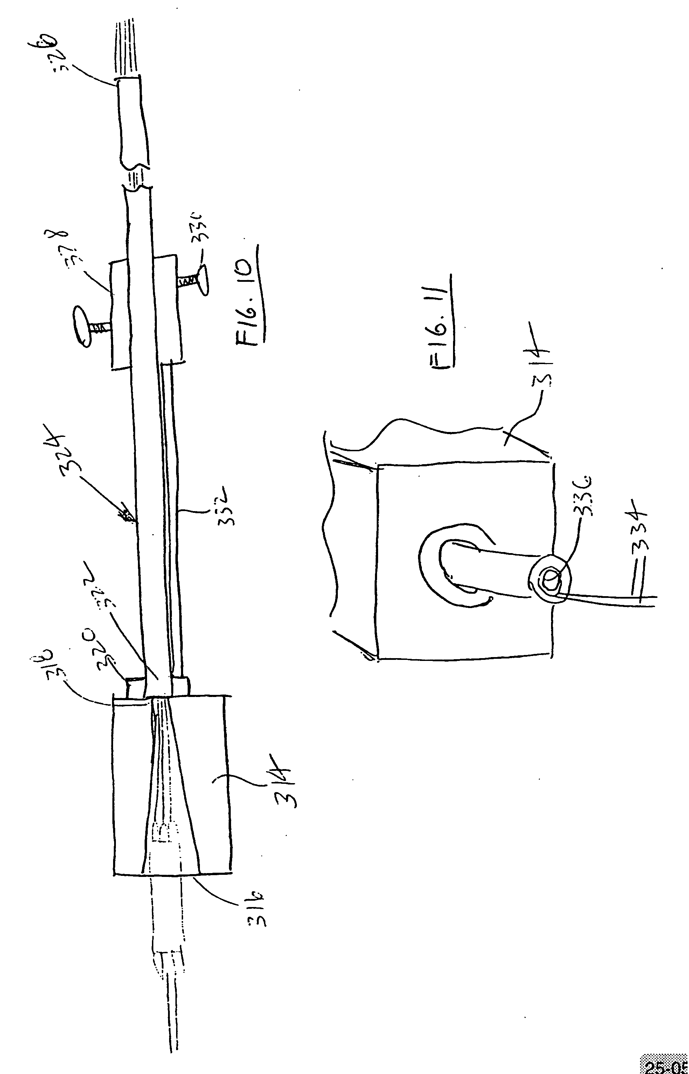

Figures 8-11 illustrate, diagrammatically, another technique and apparatus for compressing the stent and loading it into the distal end of the sleeve of the delivery device. In this technique, one or more wires, such as slender, superelastic nitinol wires, may be attached to the rhombi or diamonds, at the proximal end of the stent. Figure 8 shows two such wires 310, 312. The wire 310 is passed through a pair of diametrically opposite diamonds 308a, 308b and another wire 312 may be passed through another pair of diametrically opposite diamonds 308c, 308d. The portions of the wires 310, 312 that span the cross-section of the stent intersect at a point P located at or near the longitudinal axis of the stent. The free tails of the wires 310, 312 may be wrapped together, as with tape, and threaded through a funnel-like constrictor 314. The constrictor has an enlarged inlet end 316 and a narrowed outlet end 318 that opens into a fitting 320 adapted to engage the distal end 322 of the sleeve 324 into which the stent is to be loaded. The tails of the wires 310, 312 are passed through the sleeve 324 and exit out of the proximal end 326 of the sleeve. The device includes a clamp 328 that is separable to receive the sleeve 324. The clamp may be of a two-part construction having thumb screws 330 to enable the clamp parts to be secured together to clamp the sleeve 324. The clamp 328 is in a fixed position with respect to the constrictor 314, as by securing them to a common frame (not shown). The device also includes a trough-like support 332 that extends between the clamp 328 and the fitting 320 to support the portion of the sleeve 324 that extends between those components. With the system set-up as illustrated in FIG. 10, the stent can be drawn through the constrictor 314 and into the lumen of the sleeve 324 simply by pulling on the tails of the wires 310, 312. In this embodiment, the sleeve 324 may be transparent or translucent sufficiently to enable the stent to be visually observed as it advances into the distal end of the sleeve 324. Under such visual observation, the stent may be drawn into the distal end of the sleeve 324 to the precise location desired. The pull wires 310, 312 then may be withdrawn by pulling on one of the tails of each of the wires.

In a modification of the foregoing, an additional retraction wire 334 can be employed to enable withdrawal of the pull wires out of the distal end of the sleeve 324. In that embodiment, a retraction wire 334, constructed similarly to the pull wires 310, 312 is inserted through the distal end of the stent, out through the proximal end, and is looped about the crossed region P of the pull wires 310, 312, the end of the retraction wire 334 being passed back out through the distal end of the stent. In this embodiment, after the stent has been positioned at the intended location within the sleeve 324, the pull wires 310, 312 are withdrawn by pulling on the retraction wire in a distal direction. The retraction wire 334, being looped about the crossed pull wires, draws the pull wires, at their bight, through the sleeve 324, the stent, and the constrictor 314.

It may be desirable to provide internal support for the stent, as the stent is drawn through the constrictor. To that end, a polyimide support tube 336 can be inserted into the distal end of the stent as the stent is advanced into the constrictor. The polyimide tube is sufficiently stiff to provide luminal support for the stent as the stent is constricted and compressed to its smaller diameter. Additionally, after the stent has been loaded into the distal end of the sleeve 324, the presence of the inner support tube 336 assures that a guidewire can be passed through the sleeve 324 and the stent in order to facilitate navigation of the delivery device through the patient's vasculature. It may be noted that the polyimide tube 336, although providing internal support for the stent, nevertheless enables the retraction wire 334 as well as the pull wires 310, 312 to slide between the inner lumen of the stent and the support tube 336 to permit their removal.

### Industrial Applicability

The invention contributes to the art of stents by providing improvements in methods of loading a stent into a sleeve of a delivery system in a way which can be controlled by a data processor. Such automation furthers the objectives of consistency and quality control. Data for particular designs of stent and sleeve can be stored in the data processor, with the corresponding parameters, especially the limiting load on the pulling system, in order that the data processor shall stop pulling when the limit load is exceeded. Thus, when a batch of stents is to be loaded, the data processor will drive the system in accordance with a constant program using the stored data.

The system described above has been found to be able to handle consistently and reliably stents of a relatively large diameter too large for manual loading. Sleeving the stent in a gaseous environment furthers the object of sterility. The invention allows ready adaptation, to enable sleeving at any desired non-ambient temperature.

## Claims

1. A method of installing a stent in a sleeve, comprising the steps of
i) coupling the stent, in an expanded configuration of the stent, to an adaptor
ii) drawing the adaptor lengthwise within the sleeve, to pull the stent into the sleeve, and
iii) de-coupling the adaptor from the stent once the stent is inside
and **characterized by** the steps of
iv) coupling the distal end of the stent to the adaptor
v) pulling the stent into the proximal end of the sleeve, and the through the full length of the sleeve till the distal end of the stent is at the distal end of the sleeve; and
vi) incorporating the sleeve and stent in a stent delivery system with the stent at the distal end of the delivery system where it is releasable by a proximal withdrawal of the sleeve relative to the stent.

2. Method according to claim 1, including the step of coupling the adaptor to the stent at a plurality of push points spaced around the circumference of the leading end of the stent.

3. Method according to claim 2, including the step of providing the adaptor as a plurality of strands, each to engage with different ones of the push points.

4. Method according to claim 3, wherein each of the strands is a pull string, and each of the pull strings is associated with the push points in one-to-one correspondence.

5. Method according to claim 3, wherein each of the strands is a pull string which is looped around a pair of push points, the members of the pair being diametrically apart on the leading end of the stent.

6. Method according to claim 3, 4 or 5, including the step of pulling the strands off the stent from a point beyond the trailing end of the stent, to de-couple the adaptor from the stent once the stent is inside the sleeve.

7. Method according to claim 6, including the step of threading a release string around each of the adaptor strands, whereby pulling on the release string pulls all the strands off the leading end of the stent.

8. Method according to any one of claims 1 to 5, including the step of pushing the strands off the stent from a point beyond the leading end of the stent.

9. Method according to any one of claims 1 to 8, including the step of sensing a load imposed on the stent when drawing the stent into the sleeve, and arresting the drawing movement when a pre-set limit load is reached.

10. Method according to any one of claims 1 to 9, including the step of defining an end position of the stent relative to the sleeve, sensing the approach of the stent to said end position, and arresting the drawing movement when the end position is sensed.

11. Method according to any one of claims 1 to 10, including the step of maintaining the stent at a temperature other than ambient temperature; during the drawing step.

12. Method according to any one of claims 1 to 11, including the step of pulling the stent down a funnel into the sleeve.

13. Method according to claim 12, including the step of oscillating the funnel along its long axis, to facilitate progress of the stent towards the narrow end of the funnel.

14. Method according to any one of claims 1 to 13, including the step of restraining the sleeve at a stent-receiving end thereof, so that friction forces between the stent and the sleeve, as the stent is introduced into the sleeve, impose on the sleeve an axial tensile stress.

## Patentansprüche

1. Verfahren zum Installieren eines Stents in einer Hülle, umfassend die Schritte:
i) Koppeln des Stents in einer expandierten Konfiguration des Stents mit einem Adapter,
ii) Ziehen des Adapters in Längsrichtung innerhalb der Hülle, um den Stent in die Hülle zu ziehen, und
iii) Entkoppeln des Adapters vom Stent, wenn der Stent innen ist,
und **gekennzeichnet durch** die Schritte:
iv) Koppeln des distalen Endes des Stents mit dem Adapter,
v) Hindurchziehen des Stents in das proximale Ende der Hülle und über die volle Länge der Hülle, bis das distale Ende des Stents am distalen Ende der Hülle liegt; und
vi) Inkorporieren der Hülle und des Stents in ein Stentzufuhrsystem mit dem Stent am distalen Ende des Zufuhrsystems, wo er **durch** ein proximales Zurückziehen der Hülle relativ zum Stent freigebbar ist.

2. Verfahren gemäß Anspruch 1, einschließlich dem Schritt des Koppelns des Adapters mit dem Stent an einer Mehrzahl von Schubpunkten, die um den Umfang des führenden Endes des Stents beabstandet sind.

3. Verfahren gemäß Anspruch 2, einschließlich dem Schritt des Bereitstellens des Adapters als eine Mehrzahl von Litzen, jede zum Eingriff mit anderen der Schubpunkte.

4. Verfahren gemäß Anspruch 3, wobei jede der Litzen ein Zugfaden ist und jeder der Zugfäden mit den Schubpunkten in einer 1:1-Entsprechung assoziiert ist.

5. Verfahren gemäß Anspruch 3, wobei jede der SträngLitzene ein Zugfaden ist, der um ein Paar von Schubpunkten herumgeschleift ist, wobei die Glieder des Paars auf dem führenden Ende des Stents diametral getrennt sind.

6. Verfahren gemäß Anspruch 3, 4 oder 5, einschließlich dem Schritt des Ziehens der Litzen aus dem Stent von einem Punkt jenseits des nachlaufenden Endes des Stents, um den Adapter vom Stent zu entkuppeln, wenn der Stent einmal innerhalb der Hülle ist.

7. Verfahren gemäß Anspruch 6, einschließlich dem Schritt des Einfädelns eines Auslösefadens um jede der Adapterlitzen, wodurch das Ziehen des Auslösefadens alle Litzen vom führenden Ende des Stents abzieht.

8. Verfahren gemäß einem der Ansprüche 1 bis 5, einschließlich dem Schritt des Schiebens der Litzen weg vom Stent von einem Punkt jenseits des führenden Endes des Stents.

9. Verfahren gemäß einem der Ansprüche 1 bis 8, einschließlich dem Schritt des Feststellens einer auf dem Stent liegenden Last, wenn der Stent in die Hülle gezogen wird, und Anhalten der ziehenden Bewegung, wenn eine vorgegebene Grenzlast erreicht wird.

10. Verfahren gemäß einem der Ansprüche 1 bis 9, einschließlich dem Schritt des Definierens einer Endposition des Stents relativ zur Hülle, Feststellen der Annäherung des Stents an die Endposition und Anhalten der ziehenden Bewegung, wenn die Endposition festgestellt wird.

11. Verfahren gemäß einem der Ansprüche 1 bis 10, einschließlich dem Schritt des Haltens des Stents bei einer anderen Temperatur als der Umgebungstemperatur während des ziehenden Schritts.

12. Verfahren gemäß einem der Ansprüche 1 bis 11, einschließlich dem Schritt des Ziehens des Stents einen Trichter hinunter in die Hülle.

13. Verfahren gemäß Anspruch 12, einschließlich dem Schritt des Oszillierens des Trichters längs einer Längsachse, um das Vorrücken des Stents zum engen Ende des Trichters zu erleichtern.

14. Verfahren gemäß einem der Ansprüche 1 bis 13, einschließlich dem Schritt des Rückhaltens der Hülle an einem stentaufnehmenden Ende derselben, so dass Reibungskräfte zwischen dem Stent und der Hülle während des Einführens des Ende in die Hülle auf die Hülle eine axiale Zugspannung ausüben.

## Revendications

1. Procédé pour placer un stent dans un manchon, comprenant les étapes consistant à :
i) coupler le stent, dans une configuration déployée du stent, à un adaptateur ;
ii) aspirer l'adaptateur dans le sens de la longueur dans le manchon, afin de tirer le stent dans le manchon, et
iii) découpler l'adaptateur du stent une fois que le stent est à l'intérieur,
et **caractérisé par** les étapes consistant à
iv) coupler l'extrémité distale du stent à l'adaptateur
v) tirer le stent dans l'extrémité proximale du manchon et sur toute la longueur du manchon jusqu'à ce que l'extrémité distale du stent se trouve au niveau de l'extrémité distale du manchon ; et
vi) incorporer le manchon et le stent dans un système de pose de stent avec le stent à l'extrémité distale du système de pose où il peut être libéré par un retrait proximal du manchon par rapport au stent.

2. Procédé selon la revendication 1, comprenant l'étape consistant à coupler l'adaptateur au stent à une pluralité de points de poussée espacés autour de la circonférence de l'extrémité d'attaque du stent.

3. Procédé selon la revendication 2, comprenant l'étape consistant à proposer l'adaptateur comme une pluralité de fils, chacun pour se mettre en prise avec les différents points des points de poussée.

4. Procédé selon la revendication 3, dans lequel chacun des fils est une ficelle de traction, et chacune des ficelles de traction est associée avec les points de poussée selon une relation de un à un.

5. Procédé selon la revendication 3, dans lequel chacun des fils est une ficelle de traction qui forme une boucle autour d'une paire de points de poussée, les éléments de la paire étant diamétralement espacés sur l'extrémité d'attaque du stent.

6. Procédé selon la revendication 3, 4 ou 5, comprenant l'étape consistant à retirer les fils du stent à partir d'un point situé au-delà de l'extrémité de fuite du stent, pour découpler l'adaptateur du stent une fois que le stent est à l'intérieur du manchon.

7. Procédé selon la revendication 6, comprenant l'étape consistant à enfiler une ficelle de dégagement autour de chacun des fils d'adaptateur, moyennant quoi le fait de tirer sur la ficelle de dégagement retire tous les fils de l'extrémité d'attaque du stent.

8. Procédé selon l'une quelconque des revendications 1 à 5, comprenant l'étape consistant à pousser les fils hors du stent à partir d'un point situé au-delà de l'extrémité d'attaque du stent.

9. Procédé selon l'une quelconque des revendications 1 à 8, comprenant l'étape consistant à détecter une charge imposée sur le stent lorsque l'on aspire le stent dans le manchon, et arrêter le mouvement d'aspiration lorsqu'une charge limite prédéterminée est atteinte.

10. Procédé selon l'une quelconque des revendications 1 à 9, comprenant l'étape consistant à définir une position d'extrémité du stent par rapport au manchon, détecter l'approche du stent vers ladite position d'extrémité, et arrêter le mouvement d'aspiration lorsque la position d'extrémité est détectée.

11. Procédé selon l'une quelconque des revendications 1 à 10, comprenant l'étape consistant à maintenir le stent à une température différente de la température ambiante, pendant l'étape d'aspiration.

12. Procédé selon l'une quelconque des revendications 1 à 11, comprenant l'étape consistant à tirer le stent vers le bas d'un entonnoir dans le manchon.

13. Procédé selon la revendication 12, comprenant l'étape consistant à faire osciller l'entonnoir le long de son axe pour faciliter la progression du stent vers l'extrémité étroite de l'entonnoir.

14. Procédé selon l'une quelconque des revendications 1 à 13, comprenant l'étape consistant à retenir le manchon dans son extrémité de réception de stent, de sorte que les forces de frottement entre le stent et le manchon, lorsque le stent est introduit dans le manchon, imposent sur le manchon une contrainte de traction axiale.
